# EUROPEAN PATENT APPLICATION

(11) **EP 1 547 541 A1**
(43) Date of publication of application: **29.06.2005**
(21) Application number: 04078413.4
(22) Date of filing: 16.12.2004
(51) Int. Cl.: A61C 1/00, A61B 17/16

(54) **Surgical device for dental use with variable structure**

(30) Priority: 22.12.2003 IT mi20032548
(71) Applicant: Corti, Guido, 40139 Bologna (IT)
(72) Inventor: Corti, Guido, 40139 Bologna (IT)
(74) Representative: De Gregori, Antonella

(57) **Abstract**

A surgical device for dental use with variable structure comprising a handle (11) from which a stem (12) extends to which a work tip (13-18) is connected in a removable manner, means (26, 27, 28) being also provided for detecting the operating position of at least one tip (13-18) with respect to an external sleeve (21) containing the stem (12) provided with a tip (13-18).

## Description

The present invention concerns a surgical device for dental use with variable structure.

In modern dentistry, to be precise in implantology and bone regeneration surgery, increasingly wide use is made of osteotomes, which are surgical instruments similar to chisels with tips of different shapes.

They are used in particular in bone remodelling and small and large-scale maxillary sinus lifting operations.

Traditional osteotomes have some limitations, however. Firstly they necessarily have to be used with the aid of a hammer which acts as a percussion member at the caudal end. In this way each individual osteotome breaks the bone.

Furthermore the hammering can certainly not be controlled in terms of intensity of the force exerted, direction and axiality, it is not a reproducible action and in this way the percentage of risk of accidental perforation of the membrane is increased.

Furthermore, the hammering is certainly not pleasant for the patients who undergo these operations and it has also been proved that hammering can cause trauma and undesired side effects.

The general aim of the present invention is therefore to produce a surgical device for dental use with variable structure which permits improvement or even elimination of the above drawbacks.

A further aim of the present invention is to produce a surgical device with an extremely simple construction suitable for differentiated multiple use.

A further aim of the invention is to provide a surgical device with accurate operation in order to safeguard the patient during its use.

These aims according to the present invention are achieved by producing a surgical device for dental use with variable structure as illustrated in claim 1.

Further characteristics of the invention are highlighted by the subsequent claims.

The characteristics and advantages of a surgical device for dental use with variable structure according to the present invention will emerge more clearly from the following description, designed to provide a nonlimiting example, referring to the attached schematic drawings in which:

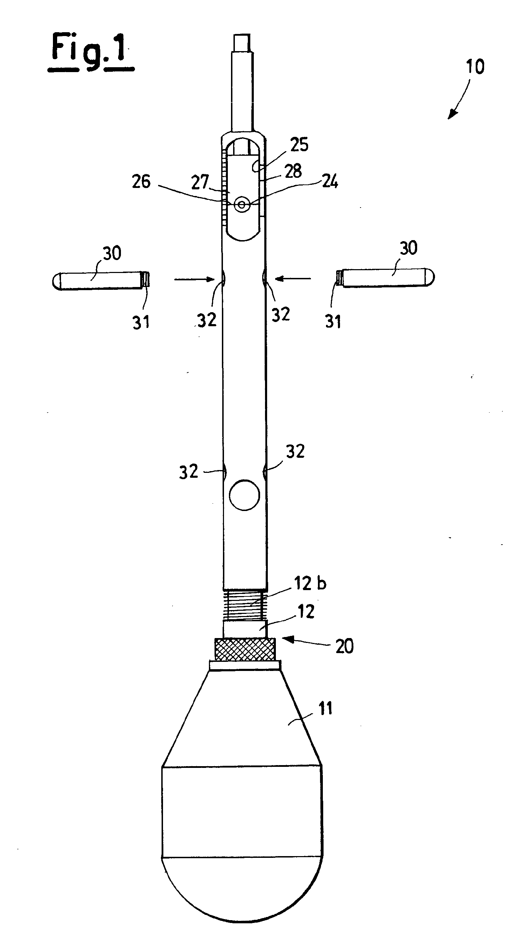

figure 1 is a front elevation view, with exploded fins, of a surgical device for dental use with variable structure according to the present invention;

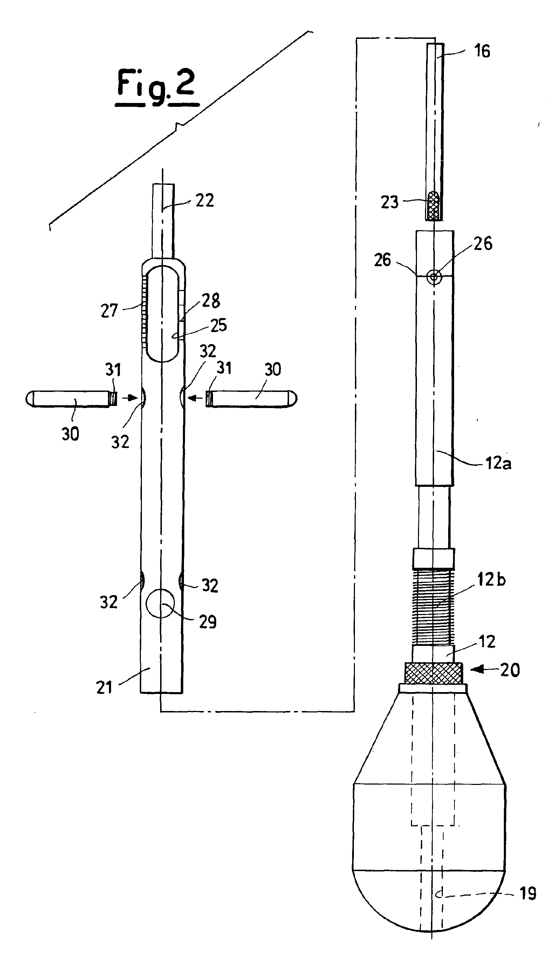

figure 2 is a front elevation view, with some elements exploded, of the device of figure 1;
figures 3a-3f are views of a certain number of tips that can be used in the device of the present invention.

With reference to the figures, a surgical device for dental use with variable structure according to the present invention is shown, indicated overall by 10.

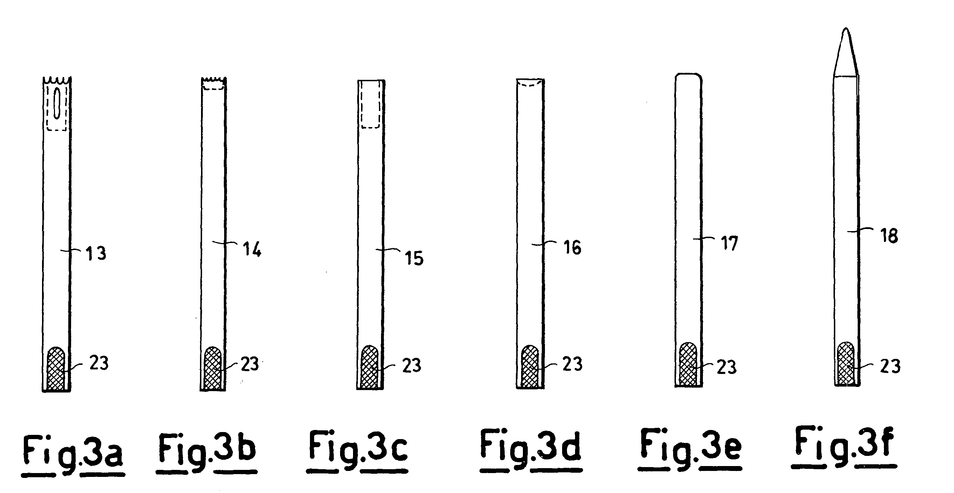

The device 10 comprises a handle 11 from which a stem 12 extends on which one of a plurality of work tips 13-18 is connected (shown in figures 3a-3f).

In particular, the stem 12 is connected to the handle 11 by means of an end screw (not shown) inserted in a hole 19 made in the handle 11. Furthermore, the handle 11 has a wider shape than the stem 12 and the remaining part of the device. A crown element 20, which acts as a stop element, is positioned in the lower part of the device and allows the operator to operate the tips 13-18 at a pre-set size, since it is possible to vary the protruding operative portion of the tip 13-18 used during work.

In fact, the stem 12, which extends integral from the crown element 20, is provided with an external threaded portion 12b on which a tubular sleeve 21 is positioned, threaded internally at one end. The tubular sleeve 21 terminates at the top in a hollow tubular end 22, with restricted diameter, in which the various tips 13-18 run and from which the operating portion of the same protrudes. Said tubular sleeve 21 acts as a containing element for the stem 12 and for a large part of the tip (13-18) in use.

The upper part 12a of the stem 12 is concave and designed to receive a milled end 23 of each tip 13-18 which permits locking thereof by means of a micro-screw 24 located in position via a screwdriver (not shown). This prevents the tips from rotating during work.

The upper part of the tubular sleeve 21, near the hollow tubular end 22, is also provided with a window 25 through which an indication 26 can be seen in the form of a reference line obtained on the upper part 12a of the stem 12. Parallel, the portions at the side of the window 25 of the upper part of the tubular sleeve 21 are provided with a pair of millimetre scales 27 and 28. A first scale 27 is graduated in millimetres while the second scale 28 is graduated in blocks of five millimetres. In this way means of identification (26, 27, 28) are provided which permit detection of the operating position of a tip 13-18 with respect to the outer sleeve 21 containing the stem 12 provided with tip 13-18.

A screw 29 inserted in a hole of the tubular sleeve 21 permits connection of said sleeve 21 to the stem 12 in a removable manner, once the reciprocal position has been selected.

Finally, fins 30 are provided with a threaded end 31, which can be inserted in threaded holes 32 obtained on the tubular sleeve 21 in a series of pre-set positions, in the example in two pairs.

The figures 3a-3f show a side elevation of a certain number of tips 13-18 which can be used in the device 10 of the present invention. In particular, a ten mm coring chisel tip 13, a two mm coring chisel tip 14, a mucotome chisel tip 15, a concave cutting chisel tip 16, a convex chisel tip 17 and a pencil chisel tip for compacting 18 are illustrated.

According to the invention a surgical device for dental use with variable structure is produced according to the present invention which considerably improves the techniques currently used in terms of ergonomy and procedure.

Furthermore, a device of this type, operation of which is evident from the figures, drastically reduces, and in some cases even eliminates, hammering, in particular in the technique of mini-lifting of the maxillary sinus.

Furthermore it should be advantageously noted that said device, due to its shape and use, allows the chisel tips to move not only in a rotating direction but also in a vertical direction.

Furthermore, it should be noted that the device is produced in surgical steel and can be used in all types of bone regeneration and remodelling. In fact, despite its dimensions, it provides easy access, also in the diatoric areas of the upper quadrants where, as is well-known, more frequent intervention is required.

The versatility of the device of the invention is guaranteed by the possibility of applying at least six different interchangeable chisel tips to one instrument only, simply and quickly.

The device also permits, with the use of one of its tips 13-18, completely manual bone coring, without the aid of rotating instruments such as surgical drills or coring devices, thus guaranteeing the clinician maximum precision and manual sensitivity.

The device also comprises, as mentioned, the stop element 20 in the form of a crown element which acts as a feed stop, in the rear part, and ensures non-interference with the work area of the tip. This means that small corrections can be made without removing the instrument from the site and maintaining an excellent field of vision, by simply acting on the crown element 20 to push out, to varying degrees, the front operative part of the tip in use.

Lastly, it should also be noted that this device enables the clinician to control, also during surgery, the work tip feed, translating it and displaying it in millimetres on the millimetre scales 27 and 28 located in the upper and therefore most visible area of the device. In this way the working depth obtained by the chisel tips can be identified at any time.

Advantageously, the two fins 30, which can be positioned as required in the holes 32, allow the operator to grip the device exerting a greater or lesser thrusting force, and they are also removable.

The removability of the sleeve 21 permits, once unscrewed, removal of the inner part for cleaning or sterilisation.

It has therefore been seen that a surgical device for dental use with variable structure according to the present invention achieves the aims previously highlighted.

The surgical device for dental use with variable structure of the present invention thus conceived can be modified and varied in numerous ways, all falling within the same inventive concept.

Furthermore, in practice any materials, dimensions and components can be used according to technical requirements.

## Claims

1. Surgical device for dental use with variable structure comprising a handle (11) from which a stem (12) extends to which a work tip (13-18) is connected in a removable manner, means (26, 27, 28) being also provided for detecting the operating position of said at least one tip (13-18) with respect to an external sleeve (21) containing the stem (12) provided with a tip (13-18).

2. Device as claimed in claim 1, **characterised in that** said identification means comprise an indication (26) obtained in an upper part (12a) of the stem (12) which aligns, as selected by the user, with a pair of millimetre-graduated scales (27, 28) arranged laterally to a window (25) obtained in said sleeve (21).

3. Device as claimed in claim 2, **characterised in that** said indication (26) is in the form of a reference line.

4. Device as claimed in claim 2, **characterised in that** a first scale (27) is graduated in millimetres while a second scale (28) is graduated in blocks of five millimetres.

5. Device as claimed in claim 1, **characterised in that** each of said tips (13-18) is connected at its lower end to an upper part (12a) of the stem (12).

6. Device as claimed in claim 1, **characterised in that** said sleeve (21) is tubular and internally threaded at one end to engage with a corresponding externally threaded portion (12b) of said stem (12).

7. Device as claimed in claim 1, **characterised in that** said sleeve (21) terminates at the top in a hollow tubular end (22), with restricted diameter with respect to a remaining tubular part of it, in which said tips (13-18) can run and be connected.

8. Device as claimed in claim 7, **characterised in that** in the lower part of said sleeve (21) a crown element (20) is positioned which can be rotated with respect to said sleeve and is integral with said stem (12), rotation of said crown element (20) pushing out or not a front portion of said at least one tip (13-18) with respect to said hollow tubular end (22).

9. Device as claimed in claim 8, **characterised in that** said sleeve (21) is made integral in a removable manner with said stem (12) by means of a locking screw (29) acting between them.

10. Device as claimed in claim 1, **characterised in that** said handle (11) has a wider shape than said stem (12) and said sleeve (21).

11. Device as claimed in claim 1, **characterised in that** said sleeve (21) is provided laterally, in a series of pre-set positions, with threaded holes (32) which receive in a freely removable manner threaded ends (31) of fins (30) for manoeuvring the device.
